# EUROPEAN PATENT APPLICATION

(11) **EP 4 183 771 A1**
(43) Date of publication of application: **24.05.2023**
(21) Application number: 21845576.4
(22) Date of filing: 20.07.2021
(51) Int. Cl.: C07C 229/28, A61K 31/195, A61P 25/04, A61P 29/00

(54) **SUSTAINED-RELEASE PHARMACEUTICAL FORMULATION OF FUSED TRICYCLIC GAMMA-AMINO ACID DERIVATIVE AND PREPARATION METHOD THEREFOR**

(30) Priority: 20.07.2020 CN 202010695946
(71) Applicant: Sichuan Haisco Pharmaceutical Co., Ltd., Sichuan Province 611130 (CN)
(72) Inventor: WANG, Qinghai, Chengdu City, Sichuan 611130 (CN); LI, Xiaoping, Chengdu City, Sichuan 611130 (CN); REN, Dong, Chengdu City, Sichuan 611130 (CN); WANG, Shunhong, Chengdu City, Sichuan 611130 (CN)
(74) Representative: Hoffmann Eitle
(86) International application number: PCT/CN2021/107286
(87) International publication number: WO 2022/017364

(57) **Abstract**

Disclosed are a sustained-release pharmaceutical formulation of a fused tricyclic γ-amino acid derivative and a preparation method therefor. The fused tricyclic γ-amino acid derivative is a compound represented by formula (I) or a stereoisomer, a solvate, a prodrug, a metabolite, a pharmaceutically acceptable salt or a eutectic crystal thereof.

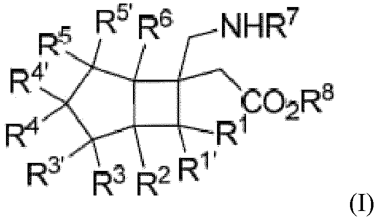

## Description

### Technical Field

The present invention relates to a sustained-release pharmaceutical formulation of a fused tricyclic γ-amino acid derivative and a preparation method therefor. The fused tricyclic γ-amino acid derivative is a compound represented by formula (I) or a stereoisomer, a solvate, a prodrug, a metabolite, a pharmaceutically acceptable salt or a eutectic crystal thereof. The present invention belongs to the technical field of biological medicine.

### Background Art

Voltage-gated calcium channels are composed of α1 subunits and auxiliary α2δ, β and γ subunits. The α2δ protein can regulate the density and voltage-dependent kinetics of the calcium channels (Felix et al. (1997) J. Neuroscience 17: 6884-6891; Klugbauer et al. (1999) J. Neuroscience 19:684-691; Hobom et al. (2000) Eur. J. Neuroscience 12:1217-1226; and Qin et al. (2002) Mol. Pharmacol. 62:485-496). It has been demonstrated that compounds having a high affinity for the voltage-dependent calcium channel subunit α2δ, such as pregabalin and gabapentin, may be effective in the treatment of pain. In mammals, the α2δ protein has four subtypes, each encoded by a different gene. α2δ subtype 1 and subtype 2 show a high affinity for pregabalin, while α2δ subtype 3 and subtype 4 do not have a significant drug-binding capacity.

However, for gabapentin, the proportion of patients with diabetic peripheral neuropathy whose pain is relieved to a great extent by using gabapentin is about 60% (Acta Neurol. Scand. 101:359-371, 2000), while for pregabalin, although it is better tolerated than gabapentin, it is less safe and may be abused or induce drug dependence in patients (Am J Health Syst Pharm. 2007; 64(14):1475-1482).

The fused tricyclic γ-amino acid derivative (formula I) has good inhibitory effects on the α2δ subunit of the calcium channels, and is associated with the endogenous inhibitory neurotransmitter γ-aminobutyric acid (GABA) related to the regulation of the brain neuronal activity. Conventional and common formulations generally need to be taken 2-3 times a day. For patients requiring long-term medication, patient compliance can be improved by taking drugs once a day. By taking drugs once a day, the maximum plasma drug concentration (Cmax) of the drugs can also be reduced, avoiding potential side effects unrelated to the therapeutic effect, and the minimum plasma drug concentration (Cmin) can also be increased, thereby increasing the drug efficacy.

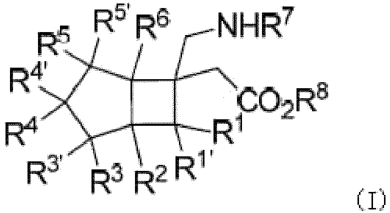

Similar to pregabalin, some specific examples of the compound of formula (I), as represented by formula (II), (III) or (IV) below, are not uniformly absorbed in the gastrointestinal (GI) tract, but are mainly absorbed in the small intestine of the human body, which indicates that the compound has a mean absorption window of no more than 6 hours. Therefore, there is a need for the development of a drug having a prolonged gastric residence time, resulting in an improved drug absorbability.

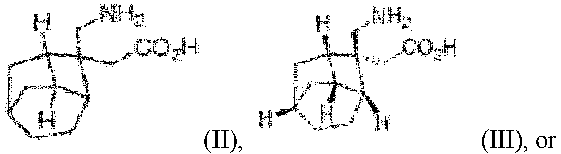

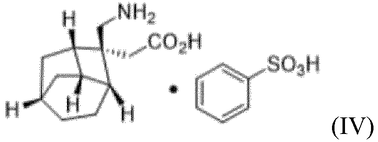

### Summary of the Invention

In order to solve the above-mentioned technical problems, the present invention provides a sustained-release pharmaceutical formulation of a fused tricyclic γ-amino acid derivative and a preparation method therefor. The fused tricyclic γ-amino acid derivative is a compound represented by formula (I), or a stereoisomer, a solvate, a prodrug, a metabolite, a pharmaceutically acceptable salt or a eutectic crystal thereof, 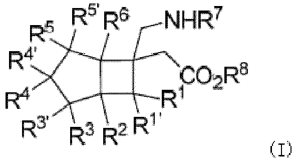
wherein R¹ and R⁴ are combined to form -(CR⁹R^{9'})n- or -CR⁹=CR^{9'}-;
R^{1'}, R², R³, R^{3'}, R^{4'}, R⁵, R^{5'}, R⁶, R⁹ or R^{9'} is each independently selected from H, F, Cl, Br, I, hydroxyl, amino, carboxyl, a carboxylate group, amido, cyano, C₁₋₆ alkyl, C₁₋₆ alkoxy, C₁₋₆ sulphanyl, C₂₋₆ alkenyl, C₂₋₆ alkynyl, 3- to 6-membered carbocyclyl or 3- to 6-membered heterocyclyl, wherein the alkyl, alkoxy, sulphanyl, alkenyl, alkynyl, carbocyclyl or heterocyclyl is optionally further substituted with 0-6 groups selected from F, Cl, Br, I, hydroxyl, amino, carboxyl, C₁₋₆ alkyl, 3- to 6-membered carbocyclyl or 3- to 6-membered heterocyclyl, and the heterocyclyl contains 1-2 heteroatoms selected from N, O or S; and n is generally selected from 1, 2 or 3, and
the pharmaceutically acceptable salt is benzene sulphonate or other salts.

In some embodiments, the fused tricyclic γ-amino acid derivative is a compound represented by formula (II).

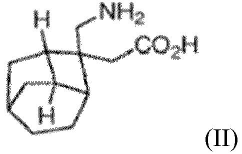

In some embodiments, the fused tricyclic γ-amino acid derivative is a compound represented by formula (III).

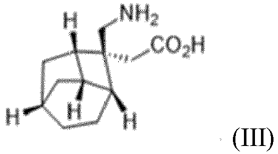

In some embodiments, the fused tricyclic γ-amino acid derivative is a compound represented by formula (IV).

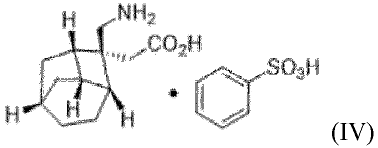

For the sustained-release pharmaceutical formulation of the present invention, the percentages by weight of the components are:

| | |
|---|---|
| Active substance | 2%-40% |
| Matrix-forming agent | 15%-50% |
| Swelling agent | 15%-70% |
| Gelling agent | 1%-45% |
| Filler | 0%-15% |
| Lubricant | 0.1%-5% |
| Total | 100% |

In some embodiments, the matrix-forming agent is selected from polyvinyl acetate, glyceryl behenate and polyvinylpyrrolidone (PVP), or a polyvinyl acetate-povidone copolymer, or any combination thereof.

Among them, polyvinylpyrrolidone is also called povidone, which is a polymer of 1-vinyl-pyrrolidin-2-one having a relative molecular mass of generally about 1 × 10³ to about 1 × 10⁷, about 2.5 × 10³ to about 3 × 10⁶ or about 1 × 10⁴ to about 1 × 10⁵.

The polyvinyl acetate-povidone copolymer can be purchased from BASF under the trade name of KOLLIDON SR^{®}, which contains about 80% of polyvinyl acetate (having a molecular weight of about 450000), about 19% of polyvinylpyrrolidone, a small amount of sodium lauryl sulphate and a small amount of silica. The matrix-forming agent can regulate the drug release to a certain extent and can be used to regulate the tablet weight.

In some embodiments, the matrix-forming agent is 20%-40% by weight.

The swelling agent comprises water-soluble or water-insoluble polymers capable of rapidly absorbing water upon contact with an aqueous medium such as gastric juice, which can increase the size of solid formulations and affect drug release rate, for example by producing a channel or forming a hydrophilic gel.

The swelling agent is 15%-70% by weight, preferably 30%-65% by weight.

In some embodiments, the swelling agent is selected from one of polyvinylpolypyrrolidone (PVPP), croscarmellose sodium, sodium carboxymethyl starch, low-substituted hydroxypropyl cellulose or polyoxyethylene, or any combination thereof.

In some embodiments, the swelling agent is polyvinylpolypyrrolidone, which can be purchased from BASF under the trade names of KOLLIDONG^{®} CL and KOLLIDONG^{®} CL-10, and can also be purchased from the ISP company under the trade names of POLYPLASDONE^{®} XL and POLYPLASDONE^{®} XL-10.

The polyvinylpolypyrrolidone is 15%-50% by weight, preferably 25%-40% by weight.

In some embodiments, the swelling agent is croscarmellose sodium, which has the same swelling effect as polyvinylpolypyrrolidone and is generally purchased from the JRS company (Germany) under the trade name of VIVASOL.

In some embodiments, the swelling agent is polyoxyethylene or a mixture of polyoxyethylene and polyvinylpolypyrrolidone in any proportion.

Polyoxyethylene can be divided into different grades according to the molecular weight and is available under the trade name of POLYOX^{®}.

When polyoxyethylene is mixed with polyvinylpolypyrrolidone for use, polyoxyethylene is generally 0%-35% by weight, preferably 5%-35% by weight, and most preferably 10%-35% by weight.

The sustained-release pharmaceutical formulation of the present invention also comprises a gelling agent, which can change the release properties of drugs. The gelling agent can be selected from one of hypromellose, sodium carboxymethyl cellulose, carbomer, xanthan gum, sodium alginate or polyoxyethylene, or any combination thereof.

In some embodiments, the gelling agent is hypromellose, which can be purchased from Dow chemistry company under the trade name of METHOCEL, divided into different grades according to the different molecular weights thereof, and selected from one of model K100LV, K4M, K15M or K100M, or any combination thereof.

Generally, hypromellose is 5%-45% by weight, preferably 10%-40% by weight.

In some embodiments, the gelling agent is carbomer, which can be purchased from the Lubrizol Corporation.

The gelling agent is 1%-35% by weight, preferably 1%-20% by weight, and most preferably 1%-10% by weight.

In some embodiments, the gelling agent is sodium alginate.

Sodium alginate is 1%-30% by weight, preferably 5%-20% by weight.

In some embodiments, the gelling agent is polyoxyethylene.

Polyoxyethylene is 1%-35% by weight, preferably 5%-35% by weight, and most preferably 10%-35% by weight.

The polyoxyethylene of the present invention can be used as both a swelling agent and a gelling agent. Upon contact with an aqueous medium such as gastric juice, polyoxyethylene can be hydrated to form a gel layer, thereby controlling the release rate of the drug.

The sustained-release pharmaceutical formulation of the present invention can also selectively contain a filler, and the filler can be selected from one of mannitol, Eudragit EPO, microcrystalline cellulose, maltodextrin, silicified microcrystalline cellulose, lactose or silica, or any combination thereof.

Generally, the filler is 0%-15% by weight.

The sustained-release pharmaceutical formulation of the present invention also comprises a lubricant that can be used to change the release properties of the drug, and the lubricant can be selected from one of magnesium stearate, talcum powder, sodium stearyl fumarate or colloidal silica, or any combination thereof.

In some embodiments, the lubricant is magnesium stearate.

Magnesium stearate is 0.1%-5% by weight, preferably 0.1%-2% by weight.

The sustained-release pharmaceutical formulation of the present invention is preferably a sustained-release tablet, which,
as desired, can also contain any suitable excipient required for tablet production, such as a diluent (microcrystalline cellulose, silicified microcrystalline cellulose, maltodextrin, mannitol, lactose, etc.) and a glidant (silica, etc.).

The sustained-release tablet of the present invention can be prepared by generally mixing the materials uniformly with a mixer by using common production technologies, and performing direct powder compression or tabletting following dry granulation. The tablet generally has a hardness of 150N-300N and has acceptable friability. The tablet die can be selected from special-shaped stamping dies such as an oval or triangular die. The tablet has a given specification enabling the tablet to immediately swell to at least 9 mm in size upon contact with gastric juice so that the tablet resides in the stomach and slowly releases the active ingredients.

The sustained-release pharmaceutical formulation obtained by the present invention rapidly absorbs water and swells upon contact with an aqueous medium, which prolongs the gastric residence time of the pharmaceutical formulation (residing in the stomach for about 3 hours to about 16 hours after oral administration) and allows for the sustained-release of the active ingredients (30% release within about 1 hour to about 4 hours, 80% release within about 12 hours to about 20 hours). Finally, the pharmaceutical formulation leaves the stomach and enters the small intestine, and the sustained-release of the active ingredients is carried on in the small intestine, thereby prolonging the release time of the active ingredients in the stomach, effectively widening the absorption window related to immediate-release administration, and achieving the effect of QD (one administration per day). The pharmaceutical formulation is suitable for one administration per day, which reduces the administration frequency and reduces the peak-to-trough ratio of the plasma concentration.

The present invention also provides a preparation method of the sustained-release tablet of the present invention, the preparation method comprising the following steps:
(1) weighing each component according to the formulation, passing the active substance and the adjuvant components except for the lubricant at amounts according to the formulation through a 40-mesh sieve, and mixing same uniformly to obtain mixture 1);
(2) passing the lubricant at an amount according to the formulation through a 40-mesh sieve, and then mixing all the materials uniformly to obtain mixture 2); and
(3) compressing the mixture 2) into tablets using suitable tabletting equipment to obtain the sustained-release tablet of the present invention.

The present invention also provides the use of the above-mentioned sustained-release pharmaceutical formulation in the preparation of a drug for treating and/or preventing pain. The pain includes: post herpetic neuralgia, trigeminal neuralgia, migraine, osteoarthritis- or arthrorheumatism-related pain, lower back pain, sciatica, toothache, pain caused by burns, pain caused by diabetic neuropathy, pain caused by chemotherapy-induced neuropathy, HIV-related neuralgia, AIDS-related neuralgia, cancer-related neuropathic pain or non-neuropathic pain, acute or chronic tension headache, post-operative pain or fibromyalgia, preferably post herpetic neuralgia, pain caused by diabetic neuropathy or fibromyalgia.

Unless stated to the contrary, the terms used in the description and claims have the following meanings.

"Percentage by weight" refers to a percentage of the mass of each component relative to the total mass of the formulation, i.e., "the weight of each component (mg)/the total weight of the formulation (mg)".

### Brief Description of the Drawings

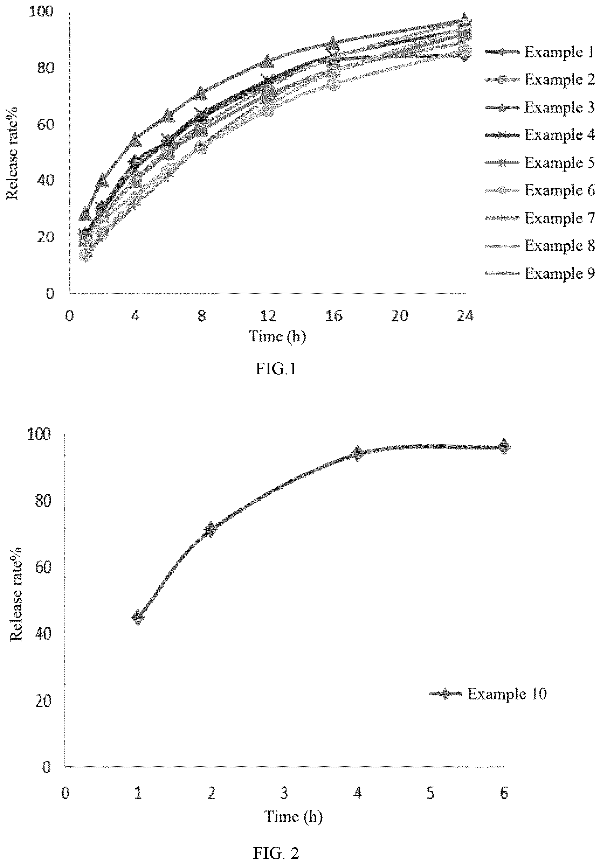

FIG. 1. *In vitro* release profiles of Examples 1-9.
FIG. 2. *In vitro* release profiles of Example 10.

### Detailed Description of Embodiments

The technical solutions of the present invention will be described in detail below in conjunction with the drawings and examples, but the protection scope of the present invention includes but is not limited thereto.

The compounds required in the examples of the present invention can be prepared according to the preparation methods disclosed in the patent applications WO 2018050046 or WO 2020011258.

### Example 1

The formulation is as follows:

| **Ingredients** | **Amount (mg)** |
|---|---|
| Compound IV | 87.79 |
| Kollidon SR | 300.21 |
| Polyvinylpolypyrrolidone | 360 |
| Polyoxyethylene N80 | 232 |
| Carbomer | 10 |
| Magnesium stearate | 10 |
| Total | 1000 |

The above components were weighed, and compound IV and the excipient components except for magnesium stearate at the amounts according to the formulation were passed through a 40-mesh sieve and then mixed uniformly to obtain mixture 1); magnesium stearate at the amount according to the formulation was passed through a 40-mesh sieve, and then all the materials were uniformly mixed to obtain mixture 2); and the mixture 2) was compressed into tablets using suitable tabletting equipment. The release profile of the compressed tablets was determined using 900 ml of hydrochloric acid medium (pH 1.2) or 900 ml of acetate buffer medium (pH 4.5).

### Example 2

The formulation is as follows:

| **Ingredients** | **Amount (mg)** |
|---|---|
| Compound IV | 87.79 |
| Kollidon SR | 258.01 |
| Polyvinylpolypyrrolidone | 290 |
| Polyoxyethylene N60K | 85 |
| Polyoxyethylene N80 | 110 |
| Carbomer | 17 |
| Magnesium stearate | 2.2 |
| Total | 850 |

The above components were weighed, and compound IV and the excipient components except for magnesium stearate at the amounts according to the formulation were passed through a 40-mesh sieve and then mixed uniformly to obtain mixture 1); magnesium stearate at the amount according to the formulation was passed through a 40-mesh sieve, and then all the materials were uniformly mixed to obtain mixture 2); and the mixture 2) was compressed into tablets using suitable tabletting equipment. The release profile of the compressed tablets was determined using 900 ml of hydrochloric acid medium (pH 1.2) or 900 ml of acetate buffer medium (pH 4.5).

### Example 3

The formulation is as follows:

| **Ingredients** | **Amount (mg)** |
|---|---|
| Compound IV | 87.79 |
| Kollidon SR | 299.71 |
| Polyvinylpolypyrrolidone | 360 |
| Polyoxyethylene N60K | 100 |
| Polyoxyethylene N12K | 130 |
| Carbomer | 20 |
| Magnesium stearate | 2.5 |
| Total | 1000 |

The above components were weighed, and compound IV and the excipient components except for magnesium stearate at the amounts according to the formulation were passed through a 40-mesh sieve and then mixed uniformly to obtain mixture 1); magnesium stearate at the amount according to the formulation was passed through a 40-mesh sieve, and then all the materials were uniformly mixed to obtain mixture 2); and the mixture 2) was compressed into tablets using suitable tabletting equipment. The release profile of the compressed tablets was determined using 900 ml of hydrochloric acid medium (pH 1.2) or 900 ml of acetate buffer medium (pH 4.5).

### Example 4

The formulation is as follows:

| **Ingredients** | **Amount (mg)** |
|---|---|
| Compound IV | 87.79 |
| Kollidon SR | 309.71 |
| Polyvinylpolypyrrolidone | 350 |
| Polyoxyethylene N60K | 100 |
| Polyoxyethylene N12K | 130 |
| Carbomer | 20 |
| Magnesium stearate | 2.5 |
| Total | 1000 |

The above components were weighed, and compound IV and the excipient components except for magnesium stearate at the amounts according to the formulation were passed through a 40-mesh sieve and then mixed uniformly to obtain mixture 1); magnesium stearate at the amount according to the formulation was passed through a 40-mesh sieve, and then all the materials were uniformly mixed to obtain mixture 2); and the mixture 2) was compressed into tablets using suitable tabletting equipment. The release profile of the compressed tablets was determined using 900 ml of hydrochloric acid medium (pH 1.2) or 900 ml of acetate buffer medium (pH 4.5).

### Example 5

The formulation is as follows:

| **Ingredients** | **Amount (mg)** |
|---|---|
| Compound IV | 87.79 |
| Kollidon SR | 300.21 |
| Polyvinylpolypyrrolidone | 280 |
| Polyoxyethylene N60K | 80 |
| Polyoxyethylene N80 | 222 |
| Carbomer | 20 |
| Magnesium stearate | 10 |
| Total | 1000 |

The above components were weighed, and compound IV and the excipient components except for magnesium stearate at the amounts according to the formulation were passed through a 40-mesh sieve and then mixed uniformly to obtain mixture 1); magnesium stearate at the amount according to the formulation was passed through a 40-mesh sieve, and then all the materials were uniformly mixed to obtain mixture 2); and the mixture 2) was compressed into tablets using suitable tabletting equipment. The release profile of the compressed tablets was determined using 900 ml of hydrochloric acid medium (pH 1.2) or 900 ml of acetate buffer medium (pH 4.5).

### Example 6

The formulation is as follows:

| **Components** | **Amount (mg)** |
|---|---|
| Compound IV | 87.79 |
| Kollidon SR | 312.21 |
| Polyvinylpolypyrrolidone | 340 |
| Sodium carboxymethyl cellulose | 150 |
| Xanthan gum | 100 |
| Magnesium stearate | 10 |
| Total | 1000 |

The components were weighed according to the formulation, and compound IV and the excipient components except for magnesium stearate at the amounts according to the formulation were passed through a 40-mesh sieve and then mixed uniformly to obtain mixture 1); magnesium stearate at the amount according to the formulation was passed through a 40-mesh sieve, and then all the materials were uniformly mixed to obtain mixture 2); and the mixture 2) was compressed into tablets using suitable tabletting equipment. The release profile of the compressed tablets was determined using 900 ml of hydrochloric acid medium (pH 1.2) or 900 ml of acetate buffer medium (pH 4.5).

### Example 7

The formulation is as follows:

| **Components** | **Amount (mg)** |
|---|---|
| Compound IV | 87.79 |
| Kollidon SR | 312.21 |
| Polyvinylpolypyrrolidone | 340 |
| Xanthan gum | 100 |
| Sodium alginate | 150 |
| Magnesium stearate | 10 |
| Total | 1000 |

The components were weighed according to the formulation, and compound IV and the excipient components except for magnesium stearate at the amounts according to the formulation were passed through a 40-mesh sieve and then mixed uniformly to obtain mixture 1); magnesium stearate at the amount according to the formulation was passed through a 40-mesh sieve, and then all the materials were uniformly mixed to obtain mixture 2); and the mixture 2) was compressed into tablets using suitable tabletting equipment. The release profile of the compressed tablets was determined using 900 ml of hydrochloric acid medium (pH 1.2) or 900 ml of acetate buffer medium (pH 4.5).

### Example 8

The formulation is as follows: (with no swelling agent and gelling agent added)

| **Components** | **Amount (mg)** |
|---|---|
| Compound IV | 87.79 |
| Kollidon SR | 185 |
| Glyceryl behenate | 70 |
| Mannitol | 75 |
| Microcrystalline cellulose | 72.21 |
| Magnesium stearate | 10 |
| Total | 500 |

The components were weighed according to the formulation, and compound IV and the excipient components except for magnesium stearate at the amounts according to the formulation were passed through a 40-mesh sieve and then mixed uniformly to obtain mixture 1); magnesium stearate at the amount according to the formulation was passed through a 40-mesh sieve, and then all the materials were uniformly mixed to obtain mixture 2); and the mixture 2) was compressed into tablets using suitable tabletting equipment. The release profile of the compressed tablets was determined using 900 ml of hydrochloric acid medium (pH 1.2) or 900 ml of acetate buffer medium (pH 4.5).

### Example 9

The formulation is as follows: (with no matrix-forming agent and swelling agent added)

| **Components** | **Amount (mg)** |
|---|---|
| Compound IV | 87.79 |
| Hypromellose | 197.2 |
| Silicified microcrystalline cellulose | 122.41 |
| Colloidal silica | 4.2 |
| Magnesium stearate | 8.4 |
| Total | 420 |

The components were weighed according to the formulation, and compound IV and the excipient components except for magnesium stearate at the amounts according to the formulation were passed through a 40-mesh sieve and then mixed uniformly to obtain mixture 1); magnesium stearate at the amount according to the formulation was passed through a 40-mesh sieve, and then all the materials were uniformly mixed to obtain mixture 2); and the mixture 2) was compressed into tablets using suitable tabletting equipment. The release profile of the compressed tablets was determined using 900 ml of hydrochloric acid medium (pH 1.2) or 900 ml of acetate buffer medium (pH 4.5).

### Example 10

The formulation is as follows: (with no matrix-forming agent and swelling agent added

| **Components** | **Amount (mg)** |
|---|---|
| Compound IV | 87.79 |
| Hypromellose | 120 |
| Mannitol | 180 |
| Silicified microcrystalline cellulose | 200.21 |
| Magnesium stearate | 12 |
| Total | 600 |

The components were weighed according to the formulation, and compound IV and the excipient components except for magnesium stearate at the amounts according to the formulation were passed through a 40-mesh sieve and then mixed uniformly to obtain mixture 1); magnesium stearate at the amount according to the formulation was passed through a 40-mesh sieve, and then all the materials were uniformly mixed to obtain mixture 2); and the mixture 2) was compressed into tablets using suitable tabletting equipment. The release profile of the compressed tablets was determined using 900 ml of hydrochloric acid medium (pH 1.2) or 900 ml of acetate buffer medium (pH 4.5).

### Example 11

The formulation is as follows:

| **Components** | **Amount (mg)** |
|---|---|
| Compound III | 66 |
| Kollidon SR | 363 |
| Polyvinylpolypyrrolidone | 270.6 |
| Polyoxyethylene N60K | 110 |
| Polyoxyethylene N12K | 154 |
| Maltodextrin | 110 |
| Carbomer | 22 |
| Magnesium stearate | 4.4 |
| Total | 1100 |

The components were weighed according to the formulation, and compound III and the excipient components except for magnesium stearate at the amounts according to the formulation were passed through a 40-mesh sieve and then mixed uniformly to obtain mixture 1); magnesium stearate at the amount according to the formulation was passed through a 40-mesh sieve, and then all the materials were uniformly mixed to obtain mixture 2); and the mixture 2) was compressed into tablets using suitable tabletting equipment. The release profile of the compressed tablets was determined using 900 ml of hydrochloric acid medium (pH 1.2) or 900 ml of acetate buffer medium (pH 4.5).

### Example 12

The formulation is as follows:

| **Components** | **Amount (mg)** |
|---|---|
| Compound IV | 154.5 |
| Kollidon SR | 285.5 |
| Polyvinylpolypyrrolidone | 286 |
| Polyoxyethylene N60K | 150 |
| Maltodextrin | 100 |
| Carbomer | 20 |
| Magnesium stearate | 4 |
| Total | 1000 |

The components were weighed according to the formulation, and compound IV and the excipient components except for magnesium stearate at the amounts according to the formulation were passed through a 40-mesh sieve and then mixed uniformly to obtain mixture 1); magnesium stearate at the amount according to the formulation was passed through a 40-mesh sieve, and then all the materials were uniformly mixed to obtain mixture 2); and the mixture 2) was compressed into tablets using suitable tabletting equipment, wherein the tablet hardness was controlled within a range of 170-220N. The release profile of the compressed tablets was determined using 900 ml of hydrochloric acid medium (pH 1.2) or 900 ml of acetate buffer medium (pH 4.5).

### Example 13

The formulation is as follows:

| **Components** | **Amount (mg)** |
|---|---|
| Compound IV | 84.3 |
| Kollidon SR | 312.7 |
| Polyvinylpolypyrrolidone | 350 |
| Polyoxyethylene N60K | 100 |
| Polyoxyethylene N12K | 130 |
| Carbomer | 20 |
| Magnesium stearate | 3 |
| Total | 1000 |

The components were weighed according to the formulation, and compound IV and the excipient components except for magnesium stearate at the amounts according to the formulation were passed through a 40-mesh sieve and then mixed uniformly to obtain mixture 1); magnesium stearate at the amount according to the formulation was passed through a 40-mesh sieve, and then all the materials were uniformly mixed to obtain mixture 2); and the mixture 2) was compressed into tablets using suitable tabletting equipment. The release profile of the compressed tablets was determined using 900 ml of hydrochloric acid medium (pH 1.2) or 900 ml of acetate buffer medium (pH 4.5).

### Swelling performance tests

Tablets were placed in a dissolution cup, and 900 ml of acetate buffer medium (pH 4.5) was added. The temperature was controlled to 37°C and the tablets were allowed to be completely immersed in the medium. The tablets were taken out at 0 h, 1 h, 2 h, 4 h, 6 h and 9 h respectively to measure the length (L), width (W) and thickness (H) thereof with a vernier caliper.

**Table 1. Swelling performance tests of Example 3 and Example 7**

| Time (h) | Example 3 | | | Example 7 | | |
|---|---|---|---|---|---|---|
| | L (mm) | W (mm) | H (mm) | L (mm) | W (mm) | H (mm) |
| Initial value | 20.68 | 10.21 | 7.90 | 20.71 | 10.25 | 7.96 |
| 1 | 21.70 | 11.44 | 9.06. | 21.94 | 11.58 | 9.22 |
| 2 | 23.41 | 12.85 | 10.47 | 23.36 | 12.69 | 10.26 |
| 4 | 24.12 | 13.72 | 11.28 | 24.57 | 13.59 | 11.19 |
| 6 | 24.83 | 14.26 | 12.05 | 24.93 | 14.03 | 11.87 |
| 9 | 25.66 | 14.53 | 12.71 | 25.48 | 14.34 | 12.33 |

The results of the swelling performance tests indicated that the drug of the present invention can swell to 9 mm or larger in size upon contact with water, and with the large swelling volume, the gastric residence time of the pharmaceutical formulation can be effectively prolonged.

### In vitro release of tablets:

1. According to "Method 2 (Paddle)" in "Dissolution and Drug Release Test" of the "Chinese Pharmacopoeia", the *in vitro* release of the self-made product was determined under the dissolution condition of 37°C and the rotational speed of 50 rpm with the dissolution medium of 900 ml of hydrochloric acid (pH 1.2) or acetate medium (pH 4.5). The results are shown in Table 2.

**Table 2. In vitro release results of Examples 1-13**

| Time (h) | Examples | | | | | | | | | | | | |
|---|---|---|---|---|---|---|---|---|---|---|---|---|---|
| | 1 | 2 | 3 | 4 | 5 | 6 | 7 | 8 | 9 | 10 | 11 | 12 | 13 |
| 1 | 21.1 | 19.0 | 28.4 | 20.4 | 19.0 | 13.8 | 13.0 | 19.0 | 18.6 | 44.8 | 22.0 | 14.2 | 17.5 |
| 2 | 30.3 | 27.4 | 40.1 | 29.6 | 27.4 | 21.5 | 20.3 | 25.8 | 27.7 | 71.3 | 31.9 | 22.4 | 26.5 |
| 4 | 46.7 | 40.0 | 54.6 | 44.3 | 40.0 | 33.8 | 31.5 | 35.3 | 40.5 | 94.0 | 45.8 | 35.3 | 39.3 |
| 6 | 53.9 | 49.9 | 63.1 | 54.3 | 49.9 | 43.7 | 41.8 | 44.4 | 51.3 | 96.2 | 56.5 | 45.7 | 49.3 |
| 8 | 62.4 | 57.9 | 71.2 | 63.6 | 57.9 | 51.8 | 52.5 | 51.8 | 59.5 | / | 64.7 | 54.5 | 56.9 |
| 12 | 74.2 | 70.4 | 82.4 | 75.5 | 70.4 | 64.9 | 68.8 | 66.5 | 73.1 | / | 77.2 | 67.3 | 68.9 |
| 16 | 82.8 | 78.8 | 88.9 | 84.2 | 78.8 | 74.2 | 79.4 | 78.6 | 83.7 | / | 85.3 | 78.0 | 77.0 |
| 24 | 84.6 | 92.3 | 97.0 | 93.7 | 92.3 | 86.2 | 89.3 | 94.3 | 96.7 | / | 90.3 | 86.7 | 84.5 |

During the *in vitro* release tests of Examples 1-13, we observed that for Example 8, which had no swelling agent and gelling agent added in the formulations, the tablet had a good sustained-release ability, but remained sunk at the bottom of the cup due to its high density. It is speculated that the tablet cannot float inside human stomach. With regard to Examples 9 and 10, which had no matrix-forming agent and swelling agent added in the formulations, Example 9 had a good sustained-release ability, but remained sunk at the bottom of the cup due to its high density. It is speculated that the tablet cannot float inside human stomach; and Example 10 had an extremely rapid rate of release and hence a poor sustained-release ability.

The drugs of Examples 1-7 and 11-13 of the present invention all can float in the cup, and their sustained-release performances were roughly consistent.

2. *In vitro* release of the above-mentioned Examples 3 and 10 was investigated using the paddle method at 75 rpm. The comparative data of the release results and the release results of the paddle method at 50 rpm were shown in Table 3.

**Table 3. Comparison of in vitro release (Paddle, 75 rpm) results of Example 3 and Example 10**

| Time (h) | Example 3 | | Example 10 | |
|---|---|---|---|---|
| | 50 rpm | 75 rpm | 50 rpm | 75 rpm |
| 1 | 28.4 | 34.2 | 44.8 | 58.6 |
| 2 | 40.1 | 46.1 | 71.3 | 82.6 |
| 4 | 54.6 | 58.0 | 94.0 | 97.8 |
| 6 | 63.1 | 67.6 | 96.2 | 96.3 |
| 8 | 71.2 | 73.8 | / | / |
| 12 | 82.4 | 85.1 | / | / |
| 16 | 88.9 | 91.4 | / | / |
| 24 | 97.0 | 99.9 | / | / |

It can be seen from the comparative test that the release rate of Example 10 was fast-increasing with the increase of the rotational speed, and the release of the drug was substantially finished at 6 hours and the tablet did not have the expected sustained-release ability; and the drug of Example 3 of the present invention can show good release consistency under different dynamic conditions and no burst release occurred, ensuring better safety.

3. The tablet obtained in Example 3 was subjected to an *in vitro* dissolution test according to the paddle method (at 50 rpm) of the "Chinese Pharmacopoeia" using acetate buffer (pH 1.2), acetate buffer (pH 4.5) and phosphate buffer (pH 6.8) (all being 900 ml) as the dissolution media respectively while the temperature of the media was controlled to 37°C. The dissolution release results are shown in Table 4.

**Table 4. Comparison of in vitro release results of Example 3 in different dissolution media**

| Time (h) | pH 1.2/50 rpm | pH 4.5/50 rpm | pH 6.8 /50 rpm |
|---|---|---|---|
| 1 | 17.9 | 18.8 | 19.3 |
| 2 | 27.4 | 28.4 | 27.7 |
| 4 | 40.1 | 40.6 | 39.7 |
| 6 | 50.1 | 50 | 49.2 |
| 8 | 58.8 | 58.8 | 57.1 |
| 12 | 69.9 | 70.4 | 69.2 |
| 16 | 77.9 | 79.9 | 79.7 |
| 20 | 85.9 | 85.3 | 86.5 |
| 24 | 90.4 | 91.2 | 92.6 |

The comparative *in vitro* release results of the drug of Example 3 in different dissolution media showed that the drug was not sensitive to changes in the pH environment and can maintain good release consistency in environments with large pH differences.

The above-mentioned test results showed that, by using the formulation technology of the present invention, the drug can swell to 9 mm or larger in size upon contact with water, which effectively prolongs the gastric residence time of the pharmaceutical formulation. At the high-intensity rotational speed (75 rpm), the pharmaceutical formulation prepared with the formulation technology of the present invention still maintains good release consistency as compared with the case at the low-intensity rotational speed (50 rpm) and no burst release occurs, ensuring better safety. In addition, the pharmaceutical formulation of the present invention is not sensitive to changes in the gastrointestinal pH environment and maintains good release consistency in different *in vitro* dissolution media, which can effectively reduce individual differences in patients after administration.

Moreover, by using the formulation technology of the present invention, the drug can be slowly released from the formulation, which ensures that the drug has a prolonged *in vivo* absorption period.

Although specific embodiments of the present invention have been described, those skilled in the art should know that the present invention can be changed and modified in many ways without departing from the range and spirit of the present invention. Therefore, the present invention is intended to cover all these changes and modifications falling within the scope of the attached claims and their equivalents.

## Claims

1. A sustained-release pharmaceutical formulation, **characterised in that** the sustained-release pharmaceutical formulation comprises:
(i) a compound represented by formula (I), or a stereoisomer, a solvate, a prodrug, a metabolite, a pharmaceutically acceptable salt or a eutectic crystal thereof as an active substance, which is 2%-40% by weight;
(ii) optionally one or more matrix-forming agents, which is/are 15%-50% by weight;
(iii) optionally one or more swelling agents, which is/are 15%-70% by weight; and
(iv) optionally one or more gelling agents, which is/are 1%-45% by weight, wherein the structure of formula (I) is as follows: 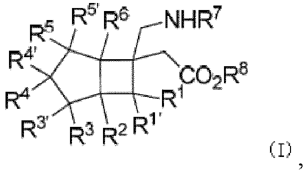
wherein R¹ and R⁴ are combined to form -(CR⁹R^{9'})n- or -CR⁹=CR^{9'}-;
R^{1'}, R², R³, R^{3'}, R^{4'}, R⁵, R^{5'}, R⁶, R⁹ or R^{9'} is each independently selected from H, F, Cl, Br, I, hydroxyl, amino, carboxyl, a carboxylate group, amido, cyano, C₁₋₆ alkyl, C₁₋₆ alkoxy, C₁₋₆ sulphanyl, C₂₋₆ alkenyl, C₂₋₆ alkynyl, 3- to 6-membered carbocyclyl or 3- to 6-membered heterocyclyl, wherein the alkyl, alkoxy, sulphanyl, alkenyl, alkynyl, carbocyclyl or heterocyclyl is optionally further substituted with 0-6 groups selected from F, Cl, Br, I, hydroxyl, amino, carboxyl, C₁₋₆ alkyl, 3- to 6-membered carbocyclyl or 3- to 6-membered heterocyclyl, and the heterocyclyl contains 1-2 heteroatoms selected from N, O or S; and n is selected from 1, 2 or 3.

2. The sustained-release pharmaceutical formulation according to claim 1, **characterised in that** the pharmaceutically acceptable salt is benzene sulphonate or other salts.

3. The sustained-release pharmaceutical formulation according to claim 1 or 2, **characterised in that** the active substance is selected from one of the following structures:

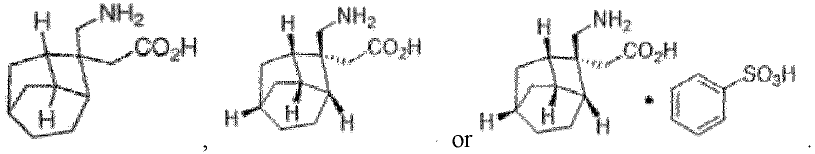

4. The sustained-release pharmaceutical formulation according to claim 3, **characterised in that** the active substance is 3%-30% by weight.

5. The sustained-release pharmaceutical formulation according to claim 3, **characterised in that** the matrix-forming agent is selected from polyvinyl acetate, glyceryl behenate, polyvinylpyrrolidone or a polyvinyl acetate-povidone copolymer, or any combination thereof.

6. The sustained-release pharmaceutical formulation according to claim 5, **characterised in that** the matrix-forming agent is 20%-40% by weight.

7. The sustained-release pharmaceutical formulation according to claim 3, **characterised in that** the swelling agent comprises water-soluble or water-insoluble polymers and is selected from one of polyvinylpolypyrrolidone, croscarmellose sodium, sodium carboxymethyl starch, low-substituted hydroxypropyl cellulose or polyoxyethylene, or any combination thereof.

8. The sustained-release pharmaceutical formulation according to claim 7, **characterised in that** the swelling agent is 30%-65% by weight.

9. The sustained-release pharmaceutical formulation according to claim 7, **characterised in that** the swelling agent is polyoxyethylene which, according to the molecular weights, can be selected from one of N80, N750, 205, N12, 1125, N60K, 301, COAGULANT or 303, or any combination thereof.

10. The sustained-release pharmaceutical formulation according to claim 7, wherein the swelling agent is polyvinylpolypyrrolidone, which is 15%-50% by weight, preferably 25%-40% by weight.

11. The sustained-release pharmaceutical formulation according to claim 7, wherein the swelling agent is a mixture of polyoxyethylene and polyvinylpolypyrrolidone in different proportions, and the polyoxyethylene is 0%-35% by weight, preferably 5%-35% by weight, and most preferably 10%-35% by weight.

12. The sustained-release pharmaceutical formulation according to claim 3, **characterised in that** the gelling agent can be selected from one of hypromellose, sodium carboxymethyl cellulose, carbomer, xanthan gum, sodium alginate or polyoxyethylene, or any combination thereof.

13. The sustained-release pharmaceutical formulation according to claim 12, **characterised in that** the gelling agent is hypromellose, which is 5%-45% by weight, preferably 10%-40% by weight.

14. The sustained-release pharmaceutical formulation according to claim 12, **characterised in that** the gelling agent is carbomer, which is 1%-35% by weight, preferably 1%-20% by weight, and most preferably 1%-10% by weight.

15. The sustained-release pharmaceutical formulation according to claim 12, **characterised in that** the gelling agent is sodium alginate, which is 1%-30% by weight, preferably 5%-20% by weight.

16. The sustained-release pharmaceutical formulation according to claim 12, **characterised in that** the gelling agent is polyoxyethylene, which is 1%-35% by weight, preferably 5%-35% by weight, and most preferably 10%-35% by weight.

17. The sustained-release pharmaceutical formulation according to claim 3, **characterised in that** the sustained-release pharmaceutical formulation can also contain a filler, wherein the filler can be selected from one of mannitol, Eudragit EPO, microcrystalline cellulose, maltodextrin, silicified microcrystalline cellulose, lactose or silica, or any combination thereof.

18. The sustained-release pharmaceutical formulation according to claim 17, **characterised in that** the filler is 0%-15% by weight.

19. The sustained-release pharmaceutical formulation according to claim 3, **characterised in that** the sustained-release pharmaceutical formulation also contains a lubricant selected from one of magnesium stearate, talcum powder, sodium stearyl fumarate or colloidal silica, or any combination thereof.

20. The sustained-release pharmaceutical formulation according to claim 19, **characterised in that** the lubricant is 0.1%-5% by weight.

21. The sustained-release pharmaceutical formulation according to claim 19, **characterised in that** the lubricant is magnesium stearate, which is 0.1%-5% by weight, preferably 0.1%-2% by weight.

22. The sustained-release pharmaceutical formulation according to claim 3, **characterised in that** the sustained-release pharmaceutical formulation is a sustained-release tablet.

23. The sustained-release pharmaceutical formulation according to claim 22, **characterised in that** the sustained-release tablet can also contain an appropriate excipient, and the excipient can be selected from one of a diluent or a glidant, or any combination thereof.

24. The sustained-release pharmaceutical formulation according to claim 23, **characterised in that** the diluent is one of microcrystalline cellulose, silicified microcrystalline cellulose, maltodextrin, mannitol or lactose, or any combination thereof, and the glidant is silica.

25. A method for preparing the sustained-release pharmaceutical formulation according to any one of claims 22-24, the method comprising the following steps:
(1) weighing each component according to the formulation, passing the active substance and the adjuvant components except for the lubricant at amounts according to the formulation through a 40-mesh sieve, and mixing same uniformly to obtain mixture 1);
(2) passing the lubricant at an amount according to the formulation through a 40-mesh sieve, and then mixing all the materials uniformly to obtain mixture 2); and
(3) compressing the mixture 2) into tablets using suitable tabletting equipment to obtain the sustained-release pharmaceutical formulation.

26. Use of the sustained-release pharmaceutical formulation according to any one of claims 1-24 in the preparation of a drug for treating and/or preventing pain.

27. The use according to claim 26, wherein the pain includes: post herpetic neuralgia, trigeminal neuralgia, migraine, osteoarthritis- or arthrorheumatism-related pain, lower back pain, sciatica, toothache, pain caused by burns, pain caused by diabetic neuropathy, pain caused by chemotherapy-induced neuropathy, HIV-related neuralgia, AIDS-related neuralgia, cancer-related neuropathic pain or non-neuropathic pain, acute or chronic tension headache, post-operative pain or fibromyalgia, preferably post herpetic neuralgia, pain caused by diabetic neuropathy or fibromyalgia.
